# EUROPEAN PATENT SPECIFICATION

(11) **EP 3 199 523 B1**
(45) Date of publication and mention of the grant of the patent: **15.08.2018**
(21) Application number: 16153393.0
(22) Date of filing: 29.01.2016
(51) Int. Cl.: C07D 211/66

(54) **THE NEW PROCESS FOR PRODUCING N-PHENYL-N-(4-PIPERIDINYL) AMIDE DERIVATIVES SUCH AS REMIFENTANIL AND CARFENTANIL**
DAS NEUE VERFAHREN ZUR HERSTELLUNG VON N-PHENYL-N- (4-PIPERIDINYL) AMID-DERIVATE WIE REMIFENTANIL UND CARFENTANIL
LE NOUVEAU PROCÉDÉ DE PRODUCTION DE N-PHÉNYL-N- (4-PIPÉRIDINYL) DES DÉRIVÉS D'AMIDES TELS QUE LE RÉMIFENTANIL ET CARFENTANIL

(43) Date of publication of application: 02.08.2017
(73) Proprietor: Bioka s. r.o., 90081 Senkvice (SK)
(72) Inventor: Kakalik, Ivan, 90081 Senkvice (SK)
(74) Representative: Belicka, Ivan

(56) References cited:
- EP-A1- 0 638 554
- EP-A1- 1 559 428
- WO-A2-2007/087164
- JÁNOS MARTON ET AL: "A Convenient Route to 4-Carboxy-4-Anilidopiperidine Esters and Acids", MOLECULES, vol. 17, no. 12, 7 March 2012 (2012-03-07) , pages 2823-2832, XP055253992, DOI: 10.3390/molecules17032823

## Description

### FIELD OF INVENTION

The present invention generally relates to a process for preparation of opiate or opioid analgesics and anesthetics, and precursors thereof. In particular, the present invention relates to process of synthetizing *N*-phenyl-*N*-(4-piperidinyl)amide derivatives such as remifentanil, carfentanil and their intermediates. The present invention relates to a preparation process with fewer steps, reduced costs, improved safety, and higher efficiency that process know in the art for producing remifentanil or carfentanil.

### BACKGROUND ART

A number of *N*-phenyl-*N*-(4-piperidinyl) amide derivatives have been described as analgesics and examples of such compound are described in U.S. patent Nos. 3998834, 5019583 and 5106983. A subclass of these *N*-phenyl-*N*-(4-piperidinyl) amide derivatives having analgesic activity are those containing the grouping: 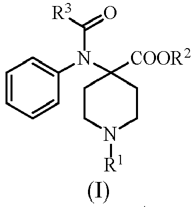 wherein R², R³ are independently methyl or ethyl and R¹ is substituted alkyl or substituted aryl such as benzyl, 2-phenylethyl, 3-methoxy-3-oxopropyl, 2-thiofuran-2-ylethyl, (4-ethyl-5-oxo-4,5-dihydro-1*H-*1,2,3,4-tetrazol-1-yl)ethyl etc.

U.S. patent no. 3998834 (and similar WO 0140184A2) specifically teaches that methyl 1-(3-methoxy-3-oxopropyl)-4-(*N*-phenylpropanamido)piperidine-4-carboxylate (remifentanil) may be prepared from the carboxylic acid (**1**) by the route outlined below (scheme 1). 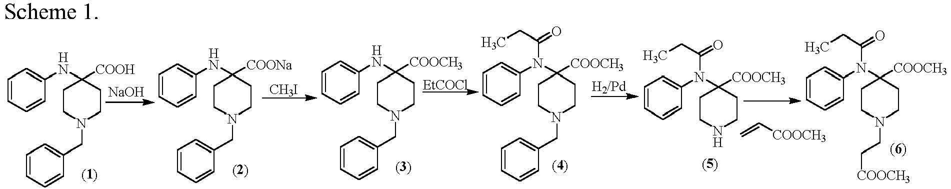 The carboxylic acid (**1**) is converted into the corresponding sodium salt (**2**), which is then reacted with methyl iodide to give the ester (**3**). Reaction of the ester (**3**) with propionic anhydride or propionyl chloride then yields the required *N*-benzyl compound (**4**), which is then converted to compound (**5**) in presence of hydrogen. In the last step compound (**5**) is N-alkylated with methyl acrylate to give remifentanil (**6**) In this reaction way used toxic reagents such as methyl iodide or dimethyl sulphate and yield of all synthetic way is very low (>40 %).

In the patent EP 0638554 (Synlett 1999, No. 12, 1923) authors solved the problem of low nucleophilicity of secondary amine nitrogen in an elegant way. They let react the amino acid (**1**) with excess propionic anhydride in the presence of trimethylamine, whereby the *in situ* generated spiro-cyclic intermediate (**7**) underwent a ring opening by methanol to the intermediate (**4**) in very good yield (schema 2).

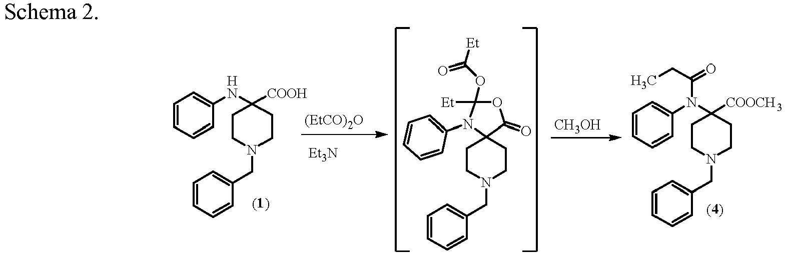

### DETAILED DESCRIPTION OF THE INVENTION

The present invention relates to an improved process for preparing *N*-phenyl-*N*-(4-piperidinyl) amide derivative (**I**) 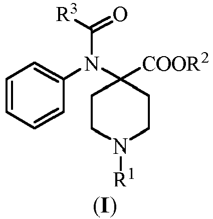 from the 4-phenylamino-4-piperidine carboxylic acid (**II**) 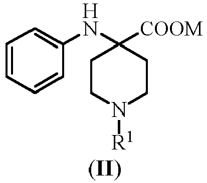 with acylating agent of formula (**IV**) in the presence of orthoester of formula (**III**) in aprotic solvent:

R-C(OR²)₃ (**III**)

R³-COX (**IV**)

wherein R², R³ are independently methyl or ethyl; R¹ is substituted alkyl or substituted aryl group such as benzyl, 2-phenylethyl, 3-methoxy-3-oxopropyl, 2-thiofuran-2-ylethyl, (4-ethyl-5-oxo-4,5-dihydro-1*H*-1,2,3,4-tetrazol-1-yl)ethyl etc.; M is hydrogen, lithium, sodium or potassium; R is hydrogen, methyl or ethyl and X is halogen atom such as chlorine, bromine and iodine.

We were surprised, that compound of formula (II) in the presence of orthoester (III) and acylating agent (IV) occur simultaneously acylation and esterification. The *N*-phenyl-*N*-(4-piperidinyl) amide derivative of formula (**I**) is prepared in one step in a high yield, which happen simultaneous acylation of the free secondary amine nitrogen and esterification of carboxylic group in structure of 4-phenylamino-4-piperidine carboxylic acid (**II**) according to the scheme 3.

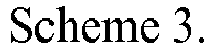

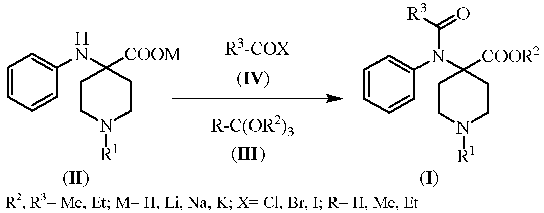

Furthermore the process has the advantage that relatively low reaction temperature may be used, short reaction times, a simple isolation procedure and avoids the use of toxic reagents (methyl iodide or dimethyl sulphate) and thus is particularly convenient for the large scale manufacture of compound of formula (**I**).

According the present invention provides a process for the preparation of *N*-phenyl-*N*-(4-piperidinyl) amide derivative of formula (**I**), which comprises reacting the salt of carboxylic acid (**II**) with acylating agent of formula (**IV**) in the presence of orthoester of formula (**III**) and aprotic solvent such as toluene, tetrahydrofuran, 1,4-dioxane, dichloromethane, ethyl acetate, isopropyl acetate, DMS, DMSO and chloroform. Suitable ortho esters of formula (**III**) are trimethyl orthoformate, triethyl orthoformate trimethyl orthoacetate, triethyl orthoacetate, trimethyl orthopropionate, triethyl orthopropionate and preferably used trimethyl orthoacetate and trimethyl orthopropionate. Suitable acylating agents (IV) are acetyl chloride, acetyl bromide, acetyl iodide, propionyl chloride, propionyl bromide, propionyl iodide and preferably used propionyl chloride.
This reaction is also conveniently carried out at a temperature within the range 15- 120°C e.g. 15-50°C and more particularly at ambient temperature or with heating e.g. 40- 60°C.
In a preferred aspect of the invention, the compound of formula (**II**) is reacted with an excess of acylating agent (**IV**) in an aprotic solvent such as dichloromethane and in the presence of an excess of the orthoester (**III**) and at reaction temperature between 15-120°C. The compound of formula (**I**) is conveniently isolated in the form of acid addition salt thereof and in particular an oxalate or hydrochloride salt thereof. The excess of the orthoester (**III**) and acylating agent (**IV**) are independently between 1.5 and 4 equivalents.

The new synthesis using simultaneous acylation and esterification are suitable for commercial manufacture of remifentanil. This new, more efficient synthesis of remifentanil includes a series of reactions shown in Scheme 4. The process starts from a commercially available lithium, sodium or potassium salt of 1-benzyl-4-(phenylamino)piperidine-4-carboxylic acid (**V**), which in the first step is debenzylated using hydrogen and a palladium catalyst to give salt of 4-phenylamino-4-piperidine carboxylic acid (**VI**). The salt of carboxylic acid (**VI**) is subsequently alkylated with methyl acrylate to yield the lithium, sodium or potassium salt of 1-(3-methoxy-3-oxopropyl)-4-phenylamino-4-piperidine carboxylic acid (**IIx**), which is then acylated with propionyl chloride in the presence of trimethyl orthoacetate or trimethyl orthopropionate to yield remifentanil (**Ia**). Products (**VI**) and (**IIx**) are not isolated. The yield of all synthetic way is about 70 %.

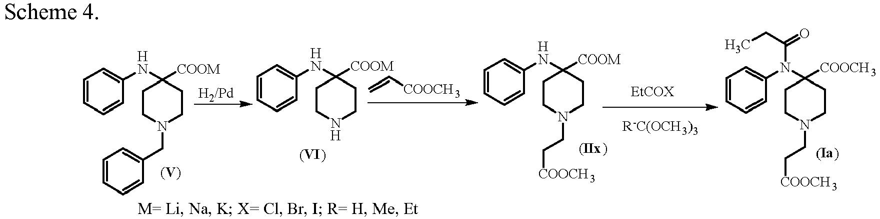

Our synthetic pathway has several advantages compared to syntheses described in US patent no. 3998834. The first advantage is the direct preparation of remifentanil by simultaneously acylation and esterification of the compound (**IIx**), which eliminates the intermediate step of preparing ester (3) from the salt of acid (**2**) (see Scheme 1), resulting in reducing the number of steps in our synthesis of fentanyl derivatives. Another advantage consists in avoids the use of toxic reagent (methyl iodide) as an alkylation agent in the synthesis of derivative (**3**) with dimethyl sulphate. The last advantage is the use of the acylation step at the end of the synthesis, avoiding thus the intramolecular rearrangement of the acyl group in the compound (**5**) (Scheme 5). 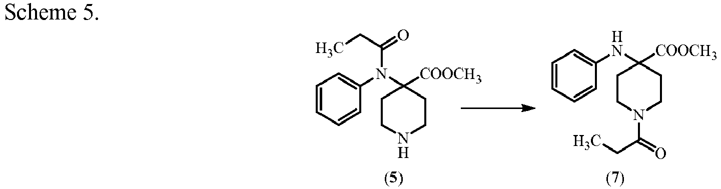 This rearrangement is responsible for decreased yields of remifentanil alkylation of the compound (**5**) (see Scheme 1). If the intermediate (VI) is alkylated in accordance with our synthetic pathway before acylation with propionyl chloride, the yield of the reaction is increased by at least 20%.

In the first step of preparation remifentanil according to the scheme 4, the benzyl protective group is removed from the compound (**V**) by reduction with hydrogen and a palladium catalyst at a temperature of 25 to 80 °C and a pressure of 1 to 10 bar. The reduction takes place in lower aliphatic alcohols such as methanol, ethanol, 1-propanol and 2-propanol and a catalyst such as 5 to 10% palladium on carbon. The reaction time is 5 to 24 hours. Reaction yields range from 90 to 100% and the HPLC purity of the product is over 98%. The resulting intermediate 4-phenylamino-4-piperidine carboxylic acid (**VI**) is alkylated in the next step with methyl acrylate to afford lithium, sodium or potassium salt of 1-(3-methoxy-3-oxopropyl)-4-phenylamino-4-piperidine carboxylic acid (**IIx**) in aliphatic alcohol such as methanol, ethanol, 1-propanol, 2-propanol as well as DMF or DMSO. The reaction temperature for alkylation ranges from 20 °C to 80 °C and the reaction time, depending on the reaction temperature, is between 20 and 50 hours. The reaction yield ranges from 70 to 98 % and the HPLC purity of the product is about 98%. In the last step, the derivative (**IIx**) is acylated and esterified with propionyl chloride in dichloromethane or chloroform in the presence of orthoester such as trimethyl orthoacetate or trimethyl orthopropionate at a temperature of 15 to 120 °C. The reaction takes 2 to 7 hours. The yields of remifentanil is range about 70 % and the HPLC purity is about 98%.

### EXAMPLES

The following examples are provided in order to more fully illustrate the present invention.

### Example 1

### Sodium salt of 4-phenylamino-4-piperidine carboxylic acid (VIa)

The 20.0 g (60,2 mmol) sodium salt of 1-benzyl-4-phenylamino-4-piperidine carboxylic acid, 2 g of 10% palladium on charcoal and 200 mL of methanol were charged into hydrogenation apparatus. The hydrogenation bottle twice flushed with hydrogen, and then the reaction mixture was stirred at 1 bar, heated at 40°C over a period of 15 hour. After reaction a mixture was cooled to room temperature and filtered. The catalyst was still washed with 30 mL of methanol and solution was evaporated to dryness. The liquid product was dried under vacuum to get 14.5 g (99.7 %) of crude product. The HPLC analysis gave 98.0 % area purity.
¹H NMR (300MHz, MeOD) δ (ppm): 1.88-1.94 (2H, m), 2.02-2.10 (2H, m), 2.80-2.91 (4H, m), 6.57 (1H, m), 6.68 (2H, d), 7.04 (2H, m)
MS: m/e =221 (M+1)⁺

### Example 2

### Potassium salt of 4-phenylamino-4-piperidine carboxylic acid (VIb)

The 10.0 g (28,7 mmol) potassium salt of 1-benzyl-4-phenylamino-4-piperidine carboxylic acid, 1 g of 10% palladium on charcoal and 200 mL of ethanol were charged into hydrogenation apparatus. The hydrogenation bottle twice flushed with hydrogen, and then the reaction mixture was stirred at 3 bar, heated at 70°C over a period of 5 hour. After reaction a mixture was cooled to room temperature and filtered. The catalyst was still washed with 20 mL of ethanol and solution was evaporated to dryness. The liquid product was dried under vacuum to get 7.1 g (96 %) of crude product. The HPLC analysis gave 97.4 % area purity.
¹H NMR (300MHz, MeOD) δ (ppm): 1.88-1.94 (2H, m), 2.02-2.10 (2H, m), 2.80-2.91 (4H, m), 6.57 (1H, m), 6.68 (2H, d), 7.04 (2H, m)
MS: m/e =221 (M+1)⁺

### Example 3

### Lithium salt of 4-phenylamino-4-piperidine carboxylic acid (VIc)

The 2.0 g (6.32 mmol) lithium salt of 1-benzyl-4-phenylamino-4-piperidine carboxylic acid, 0.2 g of 10% palladium on charcoal and 40 mL of methanol were charged into hydrogenation apparatus. The hydrogenation bottle twice flushed with hydrogen, and then the reaction mixture was stirred at 1 bar, heated at 40°C over a period of 18 hour. After reaction a mixture was cooled to room temperature and filtered. The catalyst was still washed with 10 mL of methanol and solution was evaporated to dryness. The liquid product was dried under vacuum to get 1.34 g (99 %) of crude product. The HPLC analysis gave 98.2 % area purity.
¹H NMR (300MHz, MeOD) δ (ppm): 1.88-1.94 (2H, m), 2.02-2.10 (2H, m), 2.80-2.91 (4H, m), 6.57 (1H, m), 6.68 (2H, d), 7.04 (2H, m)
MS: m/e =221 (M+1)⁺

### Example 4

### Sodium salt of 1-(3-methoxy-3-oxopropyl)-4-phenylamino-4-piperidine carboxylic acid (IIa)

The 10 g (41.3 mmol) of sodium salt of 4-phenylamino-4-piperidine carboxylic acid (VIa), 11.1 mL (123,8 mmol) of methylacrylate in 100 mL methanol is stirred during 69 hours under nitrogen atmosphere. The reaction mixture is concentrated to dryness on a rotary evaporator. The crude product is dissolved in 50 mL dichloromethane saturated and concentrated to dryness on a rotary evaporator. The crude product was dried under vacuum for 7 hours to obtain 13.4 g (98,9%) of sodium salt of 1-(3-methoxy-3-oxopropyl)-4-phenylamino-4-piperidine carboxylic acid (IIa). The HPLC analysis gave 97.4 % area purity.
¹H NMR (300MHz, MeOD) δ (ppm): 1.96-2.05 (2H, m), 2.15-2.24 (2H, m), 2.43-2.56 (4H, m), 2.62-2,72 (4H, m), 3.66 (3H, s), 6.56 (1H, m), 6.68 (2H, d), 7.03 (2H, m)
¹³C NMR, δ: 23.00 (CH₂), 24.53 (2xCH₂), 40.99 (2xCH₂N), 42.67 (CH₃O), 45.12 (CH₂N), 50.33 (C), 106.79 (2xCHₐᵣ), 108.29 (CHₐᵣ), 120.02 (2xCHₐᵣ), 138.31 (Cₐᵣ), 146.99 (COO), 173.27 (COO)
MS: m/e =307 (M+1)⁺

### Example 5

### Potassium salt of 1-(3-methoxy-3-oxopropyl)-4-phenylamino-4-piperidine carboxylic acid (IIb)

The 5 g (19.35 mmol) of pottasium salt of 4-phenylamino-4-piperidine carboxylic acid (VIb), 5.2 mL (58.05 mmol) of methylacrylate in 80 mL ethanol is stirred during 100 hours under nitrogen atmosphere. The reaction mixture is concentrated to dryness on a rotary evaporator. The crude product is dissolved in 50 mL dichloromethane saturated and concentrated to dryness on a rotary evaporator. The crude product was dried under vacuum for 4 hours to obtain 6.4 g (96.7 %) of potassium salt of 1-(3-methoxy-3-oxopropyl)-4-phenylamino-4-piperidine carboxylic acid (IIb). The HPLC analysis gave 97.4 % area purity.
¹H NMR (300MHz, MeOD) δ (ppm): 1.96-2.05 (2H, m), 2.15-2.24 (2H, m), 2.43-2.56 (4H, m), 2.62-2,72 (4H, m), 3.66 (3H, s), 6.56 (1H, m), 6.68 (2H, d), 7.03 (2H, m)
¹³C NMR, δ: 23.00 (CH₂), 24.53 (2xCH₂), 40.99 (2xCH₂N), 42.67 (CH₃O), 45.12 (CH₂N), 50.33 (C), 106.79 (2xCHₐᵣ), 108.29 (CHₐᵣ), 120.02 (2xCHₐᵣ), 138.31 (Cₐᵣ), 146.99 (COO), 173.27 (COO)
MS: m/e =307 (M+1)⁺

### Example 6

### Remifentanil: Methyl 1-(3-methoxy-3-oxopropyl)-4-(N-phenylpropanamido)piperidine-4-carboxylate (Ia)

The 2 g (15.2 mmol) of sodium salt of 1-(3-methoxy-3-oxopropyl)-4-phenylamino-4-piperidine carboxylic acid (IIa), 4.3 ml (30.45 mmol) trimethyl orthopropionate in 80 ml dichloromethane is suspended. Thereafter 5.3 ml (61 mmol) of propionyl chloride was added drop wise to the reaction mixture over a period of 10 minute. After the addition was complete, the reaction mixture is stirred under reflux during 4 hours under nitrogen atmosphere. The reaction mixture is cooled to 25°C and extracted with 40 ml of 20 % solution of sodium carbonate, than dried with sodium sulphate. The solid was filtered off and the solution was evaporated to dryness. The crude product was dissolved in 50 ml 2-propanol saturated with gaseous hydrochloric acid (15%). The product was cooled at 10°C over a period of 8 hours. The crystals was filtered and dried under vacuum for 4 hours to obtain 5.1 g (81.1 %) of methyl 1-(3-methoxy-3-oxopropyl)-4-(*N*-phenylpropanamido)-piperidine-4-carboxylate hydrochloride. The product purity was determined to be 98.8 % area by liquid chromatography.
¹H NMR (300MHz, CDCl₃) δ (ppm): 0.96 (3H, t), 1,66 (2H, m), 1.88 (2H, t), 2.28 (2H, m), 22.42 (4H, m), 2.66(4H, m), 3.62 (3H, s), 3.79 (3H, s), 7.32 (2H, m), 7.41 (3H, m)
¹³C NMR, δ: 9.15 (CH₃), 29.06 (2xCH₂), 31.98 (CH₂), 33.37(CH₂), 49.63 (2xCH₂N), 51.65 (CH₃O), 52.12 (CH₃O), 53.22 (CH₂N), 62.64 (C), 128.71 (CHₐᵣ), 129.45 (2xCHₐᵣ), 130.12 (2xCHₐᵣ), 139.32 (Cₐᵣ), 172.84 (CON), 173.94 (COO), 174.12 (COO)
MS: m/e =377 (M+1)⁺

### Example 7

### Remifentanil: Methyl 1-(3-methoxy-3-oxopropyl)-4-(N-phenylpropanamido)piperidine-4-carboxylate (Ia)

The 1 g (3.05 mmol) of potassium salt of 1-(3-methoxy-3-oxopropyl)-4-phenylamino-4-piperidine carboxylic acid (IIb), 0.76 ml (6.1 mmol) trimethyl orthoacetate in 30 ml chloroform is suspended. Thereafter 5.3 ml (6.1 mmol) of propionyl chloride was added drop wise to the reaction mixture over a period of 2 minute. After the addition was complete, the reaction mixture is stirred under reflux during 2 hours under nitrogen atmosphere. The reaction mixture is cooled to 25°C and extracted with 10 ml of 20 % solution of sodium carbonate, than dried with sodium sulphate. The solid was filtered off and the solution was evaporated to dryness. The crude product was dissolved in 15 ml 2-propanol saturated with gaseous hydrochloric acid (10%). The product was cooled at 10°C over a period of 4 hours. The crystals was filtered and dried under vacuum for 1 hours to obtain 0.78 g (62.4 %) of methyl 1-(3-methoxy-3-oxopropyl)-4-(*N*-phenylpropanamido)piperidine-4-carboxylate hydrochloride. The product purity was determined to be 97.6 % area by liquid chromatography.
¹H NMR (300MHz, CDCl₃) δ (ppm): 0.96 (3H, t), 1,66 (2H, m), 1.88 (2H, t), 2.28 (2H, m), 22.42 (4H, m), 2.66(4H, m), 3.62 (3H, s), 3.79 (3H, s), 7.32 (2H, m), 7.41 (3H, m)
¹³C NMR, δ: 9.15 (CH₃), 29.06 (2xCH₂), 31.98 (CH₂), 33.37(CH₂), 49.63 (2xCH₂N), 51.65 (CH₃O), 52.12 (CH₃O), 53.22 (CH₂N), 62.64 (C), 128.71 (CHₐᵣ), 129.45 (2xCHₐᵣ), 130.12 (2xCHₐᵣ), 139.32 (Cₐᵣ), 172.84 (CON), 173.94 (COO), 174.12 (COO)
MS: m/e =377 (M+1)⁺

### Example 8

### Carfentanil: Methyl 1-(2-phenylethyl)-4-(N-phenylpropanamido)piperidine-4-carboxylate (Ib)

The 1 g (2.90 mmol) of potassium salt 1-(2-phenylethyl)-4-phenylamino-4-piperidine carboxylic acid (IIb), 0.82 ml (5.77 mmol) trimethyl orthopropionate in 40 ml dichloromethane is suspended. Thereafter 0.76 ml (8.66 mmol) of propionyl chloride was added drop wise to the reaction mixture over a period of 4 minute. After the addition was complete, the reaction mixture is stirred under reflux during 5 hours under nitrogen atmosphere. The reaction mixture is cooled to 25°C and extracted with 10 ml of 10 % solution of sodium hydroxide, than dried with sodium sulphate. The solid was filtered off and the solution was evaporated to dryness. The resulting residue was dissolved in 25 ml methylisobutylketone and oxalic acid dihydrate (0.38 g, 3 mmol) in methylisobutylketone (15 mL) was added. The product was cooled at 10°C over a period of 3 hours. The crystals was filtered and dried under vacuum for 3 hours to obtain 1.02 g (73 %) of methyl 1-(2-phenylethyl)-4-(*N*-phenylpropanamido)piperidine-4-carboxylate oxalate. The product purity was determined to be 98.7 % area by liquid chromatography.
¹H-NMR (300MHz, DMSO-d6) δ (ppm): 0.80 (3H, t), 1.77 (2H, q), 1.83 (2H, m), 2.21 (2H, m), 2.85 (2H, m), 2.97 (2H, m), (2H, m), 3.06 (2H, m), 3,25 (2H, m), 3,70 (3H, s), 7.12-7,60 (10H, m)
¹³C-NMR (CDCl3) δ: 9.0 (CH₃), 28.2 (CH₂), 30.0 (2xCH₂), 30.3 (CH₂), 48.6 (2xCH₂), 52.3 (CH₂N), 56.6 (OCH3), 60.1 (C), 126.6 (CHₐᵣ), 128.5 (2xCHₐᵣ), 128.6 (CHₐᵣ), 129.0 (2xCHₐᵣ), 130.0 (2xCHₐᵣ), 130.1 (2xCHₐᵣ), 137.2 (Cₐᵣ), 138.1 (Cₐᵣ), 172.8 (CON), 164.3 ((COOH)2), 173.4 (COO)
MS: m/e =395 (M+1)⁺

### Example 9

### Methyl 1-benzyl-4-(N-phenylpropanamido)piperidine-4-carboxylate (Ic)

The 14.25 g (42.86 mmol) of sodium salt of 1-benzyl-4-phenylamino-4-piperidine carboxylic acid, 9.14 ml (64.3 mmol) trimethyl orthopropionate in 140 ml dichloromethane is suspended. Thereafter 11.2 ml (128.6 mmol) of propionyl chloride was added drop wise to the reaction mixture over a period of 2 minute. After the addition was complete, the reaction mixture is stirred under reflux during 1 hours under nitrogen atmosphere. The reaction mixture is cooled to 25°C and extracted with 80 ml of 25 % solution of sodium carbonate, than dried with sodium sulphate. The solid was filtered off and the solution was evaporated to dryness. The resulting residue was dissolved in 145 ml methanol and 3.85 g (42.86 mmol) oxalic acid was added. The product was cooled at 10°C over a period of 3 hours. The crystals was filtered and dried under vacuum for 2 hours to obtain 13.4 g (66.4 %) of methyl 1-benzyl-4-(*N-*phenylpropanamido)piperidine-4-carboxylate oxalate. The product purity was determined to be 99.7 % area by liquid chromatography.
¹H-NMR (300MHz, DMSO) δ (ppm): 0.80 (3H, t), 1.71-1.85 (4H, m), 2.18-2.23 (2H, m), 3.02-3.15 (2H, m), 3.70 (3H, s), 4.06 (2H, s), 7.26-7.51 (10H, m)
¹³C-) NMR (DMSO) δ: 9.05 (CH₃), 28.24 (CH₂), 30.38 (2xCH₂), 48.25 (2xCH₂), 52.22 (CH₃O), 59.26 (C), 60.23 (CH₂), 128.56 (2xCHₐᵣ), 128.85 (CHₐᵣ), 128.91 (CHₐᵣ), 129.53 (2xCHₐᵣ), 130.32 (2xCHₐᵣ), 130.73 (2xCHₐᵣ), 131.45 (Cₐᵣ), 138.4 (Cₐᵣ), 163.71 ((COOH)2), 172.48 (CON), 173.16 (COO)
MS: m/e =381 (M+1)⁺

### Example 10

### Methyl 1-benzyl-4-(N-phenylpropanamido)piperidine-4-carboxylate (Ic)

The 1 g (3.16 mmol) of lithium salt of 1-benzyl-4-phenylamino-4-piperidine carboxylic acid, 0.75 ml (6.01 mmol) trimethyl orthoacetate in 15 ml chloroform is suspended. Thereafter 1.13 ml (12.6 mmol) of propionyl bromide was added drop wise to the reaction mixture over a period of 2 minute. After the addition was complete, the reaction mixture is stirred under reflux during 2 hours under nitrogen atmosphere. The reaction mixture is cooled to 25°C and extracted with 10 ml of 25 % solution of sodium carbonate, than dried with sodium sulphate. The solid was filtered off and the solution was evaporated to dryness. The resulting residue was dissolved in 15 ml 2-propanol and 0.27 g (3.01 mmol) oxalic acid was added. The product was cooled at 15°C over a period of 3 hours. The crystals was filtered and dried under vacuum for 2 hours to obtain 1.1 g (78.5 %) of methyl 1-benzyl-4-(*N*-phenylpropanamido)piperidine-4-carboxylate oxalate. The product purity was determined to be 98.31 % area by liquid chromatography.
¹H-NMR (300MHz, DMSO) δ (ppm): 0.80 (3H, t), 1.71-1.85 (4H, m), 2.18-2.23 (2H, m), 3.02-3.15 (2H, m), 3.70 (3H, s), 4.06 (2H, s), 7.26-7.51 (10H, m)
¹³C-) NMR (DMSO) δ: 9.05 (CH₃), 28.24 (CH₂), 30.38 (2xCH₂), 48.25 (2xCH₂), 52.22 (CH₃O), 59.26 (C), 60.23 (CH₂), 128.56 (2xCHₐᵣ), 128.85 (CHₐᵣ), 128.91 (CHₐᵣ), 129.53 (2xCHₐᵣ), 130.32 (2xCHₐᵣ), 130.73 (2xCHₐᵣ), 131.45 (Cₐᵣ), 138.4 (Cₐᵣ), 163.71 ((COOH)₂), 172.48 (CON), 173.16 (COO)
MS: m/e =381 (M+1)⁺

### Example 11

### Ethyl 1-benzyl-4-(N-phenylpropanamido)piperidine-4-carboxylate (Id)

The 2 g (6.02 mmol) of sodium salt of 1-benzyl-4-phenylamino-4-piperidine carboxylic acid, 1.67 ml (90.25 mmol) triethyl orthopropionate in 60 ml ethyl acetate is suspended. Thereafter 11.2 ml (12.86 mmol) of propionyl chloride was added drop wise to the reaction mixture over a period of 10 minute. After the addition was complete, the reaction mixture is stirred under reflux during 3 hours under nitrogen atmosphere. The reaction mixture is cooled to 25°C and extracted with 80 ml of 25 % solution of sodium carbonate, than dried with sodium sulphate. The solid was filtered off and the solution was evaporated to dryness. The resulting residue was dissolved in 30 ml 2-propanol and 0.54 g (6.02 mmol) oxalic acid was added. The product was cooled at 10°C over a period of 3 hours. The crystals was filtered and dried under vacuum for 2 hours to obtain 2.45 g (84.5 %) of ethyl 1-benzyl-4-(*N*-phenylpropanamido)piperidine-4-carboxylate oxalate. The product purity was determined to be 99.8 % area by liquid chromatography.
¹H-NMR (300MHz, DMSO) δ (ppm): 0.91 (3H, t), 1.24 (3H, t) 1.70-1.80 (4H, m), 2.16-2.18 (2H, m), 3.00-3.10 (2H, m), 4.06 (2H, s), 4.23 (2H, q), 7.25-7.55 (10H, m)
¹³C-) NMR (DMSO) δ: 9.05 (CH₃), 13,62 (CH₃), 28.41 (CH₂), 30.62 (2xCH₂), 48.18 (2xCH₂), 59.26 (C), 60.23 (CH₂), 62.17 (CH₂O), 128.66 (2xCHₐᵣ), 128.75 (CHₐᵣ), 129.1 (CHₐᵣ), 129.83 (2xCHₐᵣ), 130.52 (2xCHₐᵣ), 131.11 (2xCHₐᵣ), 131.52 (Cₐᵣ), 138.81 (Cₐᵣ), 164.10 ((COOH)₂), 172.51 (CON), 172.98 (COO)
MS: m/e =394 (M+1)⁺

### Example 12

### Methyl 1-benzyl-4-(N-phenylethanamido)piperidine-4-carboxylate (Ie)

The 1 g (3.16 mmol) of lithium salt of 1-benzyl-4-phenylamino-4-piperidine carboxylic acid, 0.75 ml (6.01 mmol) trimethyl orthoacetate in 15 ml chloroform is suspended. Thereafter 0.86 ml (12.04 mmol) of acetyl chloride was added drop wise to the reaction mixture over a period of 2 minute. After the addition was complete, the reaction mixture is stirred under reflux during 3 hours under nitrogen atmosphere. The reaction mixture is cooled to 25°C and extracted with 10 ml of 25 % solution of sodium carbonate, than dried with sodium sulphate. The solid was filtered off and the solution was evaporated to dryness. The resulting residue was dissolved in 15 ml 2-propanol and 0.27 g (3.01 mmol) oxalic acid was added. The product was cooled at 15°C over a period of 5 hours. The crystals was filtered and dried under vacuum for 2 hours to obtain 1.1 g (78.5 %) of methyl 1-benzyl-4-(*N*-phenylethanamido)piperidine-4-carboxylate oxalate. The product purity was determined to be 98.31 % area by liquid chromatography.
¹H-NMR (300MHz, DMSO) δ (ppm):1.78-2.2 (7H, m), 2.25-2.28 (2H, m), 3.00-3.20 (2H, m), 3.70 (3H, s), 4.12 (2H, s), 7.20-7.55 (10H, m)
¹³C-) NMR (DMSO) δ: 20.63(CH₃), 28.16 (CH₂), 30.41 (2xCH₂), 48.20 (2xCH₂), 52.31 (CH₃O), 59.28 (C), 60.23 (CH₂), 128.56 (2xCHₐᵣ), 128.85 (CHₐᵣ), 128.91 (CHₐᵣ), 129.53 (2xCHₐᵣ), 130.32 (2xCHₐᵣ), 130.81 (2xCHₐᵣ), 131.50 (Cₐᵣ), 138.45 (Cₐᵣ), 163.70 ((COOH)₂), 172.14 (CON), 172.98 (COO)
MS: m/e =367 (M+1)⁺

### INDUSTRIAL UTILITY

The invention has industrial utility in the production of the pharmaceutical substances of remifentanil or carfentanil and of an injection forms containing these substances.

## Claims

1. A process for the preparation of the compound of formula (**I**): 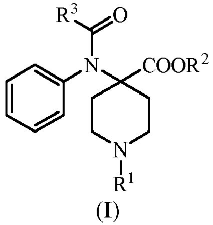 which comprises reacting a compound of formula (**II**): 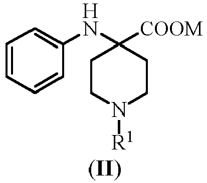 with acylating agent of formula (**IV**) in the presence of orthoester of formula (**III**) in aprotic solvent:
R-C(OR²)₃ **III**)
R³-COX (**IV**)
wherein R², R³ are independently methyl or ethyl; M is hydrogen, lithium, sodium or potassium; R is hydrogen, methyl or ethyl and X is halogen atom such as chlorine, bromine and iodine; R¹ is substituted alkyl or substituted aryl group such as benzyl, 2-phenylethyl, 3-methoxy-3-oxopropyl, 2-thiofuran-2-ylethyl, (4-ethyl-5-oxo-4,5-dihydro-1*H*-1,2,3,4-tetrazol-1-yl)ethyl.

2. A process according to claim 1 **characterised in that** aprotic solvent is toluene, tetrahydrofuran, 1,4-dioxane, dichloromethane, ethyl acetate, isopropyl acetate, DMS, DMSO and chloroform.

3. A process according to claim 1 **characterised in that** acylating agent is acetyl chloride, acetyl bromide, acetyl iodide, propionyl chloride, propionyl bromide, propionyl iodide

4. A process according to claim 1 **characterised in that** orthoester is trimethyl orthoformate, triethyl orthoformate, trimethyl orthoacetate, triethyl orthoacetate, trimethyl orthopropionate, triethyl orthopropionate.

5. A process according to claim 1 **characterised in that** the reaction is carried out at a temperature within the range 15- 120°C.

6. A process according to claim 1 **characterised in that** the compound of formula (**I**) is isolated in the form of acid addition salt thereof.

7. A process for the preparation of remifentanil (**Ia**) according enclosed figure comprised from steps:
a) catalytic debenzylation of lithium, sodium or potassium salt of 1-benzyl-4-(phenylamino)piperidine-4-carboxylic acid (**V**) with palladium on charcoal
b) alkylation of lithium, sodium or potassium salt of 4-phenylamino-4-piperidine carboxylic acid (**VI**) with methyl acrylate
c) acylation of lithium, sodium or potassium salt of 1-(3-methoxy-3-oxopropyl)-4-phenylamino-4-piperidine carboxylic acid (**IIx**) with propionyl chloride, propionyl bromide or propionyl iodide in the presence of trimethyl orthoacetate or trimethyl orthopropionate in dichloromethane and final remifentanil is isolated in the form of oxalate or hydrochloride salt.

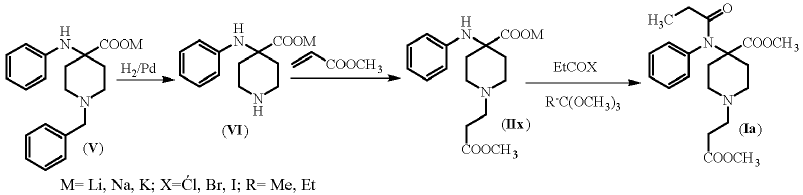

8. A process according to claim 7 **characterised in that** the benzyl protective group removed from the compound (**V**) by reduction with hydrogen and a palladium catalyst at a temperature 25 to 80 °C and a pressure of 1 to 10 bar in aliphatic alcohol.

9. A process according to claim 7 **characterised in that** alkylation of salt of 4-phenylamino-4-piperidine carboxylic acid (**VI**) with methyl acrylate is performed at a temperature within the range 25- 80 °C in aliphatic alcohol.

10. A process according to claim 8 and 9 **characterised in that** aliphatic alcohol is methanol, ethanol, 1-propanol and 2-propanol.

## Patentansprüche

1. Verfahren zur Herstellung einer Verbindung mit der Formel (**I**): 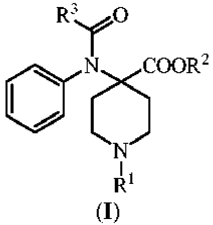 dass erfasst die Reaktion einer Verbindung mit der Formel (**II**): 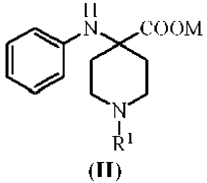 mit einem Acylierungsmittel mit der Formel (**IV**) in Anwesenheit eines Orthoesters mit der Formel (**III**) in einem aprotischen Lösungsmittel:
R-C(OR²)₃ (**III**)
R³-COX (**IV**)
wobei R², R³ sind unabhängig voneinander Methyl oder Ethyl; M ist Wasserstoff, Lithium, Natrium und Kalium; R ist Wasserstoff, Methyl oder Ethyl und X ist ein Halogen, wie Chlor, Brom und Iod; R¹bedeutet substituiertes Alkyl Gruppe oder substituiertes Aryl Gruppe, wie Benzyl, 2-phenylethyl, 3-methoxy-3-oxopropyl, 2-thiofuran-2-ylethyl, (4-ethyl-5-oxo-4,5-dihydro-1***H**-*1,2,3,4-tetrazol-1-yl)ethyl.

2. Verfahren nach Anspruch 1, **dadurch gekennzeichnet, dass** ein aprotische Lösungsmittel Toluol, Tetrahydrofuran, 1,4-Dioxan, Dichlormethan, Ethylacetat, Isopropylacetat, DMS, Chloroform und DMSO ist.

3. Verfahren nach Anspruch 1, **dadurch gekennzeichnet, dass** ein Acylierungsmittel Acetylchlorid, Acetylbromid, Acetyljodid, Propionylchlorid, Propionylbromid, Propionyljodid ist

4. Verfahren nach Anspruch 1, **dadurch gekennzeichnet, dass** Orthoester Orthoameisensäuretrimethylester, Triethylorthoformiat, Trimethylorthoacetat, Triethylorthoacetat, Trimethylorthopropionat, Triethylorthopropionat ist.

5. Verfahren nach Anspruch 1, **dadurch gekennzeichnet, dass** die Reaktion wird bei einer Temperatur innerhalb des Bereichs 15 bis 120 °C durchgeführt ist.

6. Verfahren nach Anspruch 1, **dadurch gekennzeichnet, dass** die Verbindung mit der Formel (I) als Säureadditionssalz isoliert ist.

7. Das Verfahren für die Herstellung von Remifentanil (**Ia**) nach beigefügtes Bild, bestehend aus Schritten:
a) katalytische Debenzylierung Lithium-, Natrium- oder Kaliumsalz der 1-benzyl-4-(phenylamino)piperidin-4 Carbonsäure (V) mit Palladium auf Aktivkohle
b) Alkylierung eines Lithium-, Natrium- oder Kaliumsalz der 4-phenylamino-4-piperidin Carbonsäure (VI) mit Methylacrylat
c) Acylierung des Lithium-, Natrium- oder Kaliumsalz der 1-(3-methoxy-3-oxopropyl)-4-phenylamino-4-piperidin Carbonsäure (**IIx**) mit Propionylchlorid, Propionylbromid oder Propionyljodid in Anwesenheit von Trimethylorthoacetat oder Trimethylorthopropionat in Dichlormethan und resultierend Remifentanil als Oxalat- oder Hydrochlorid-Salz isoliert ist.

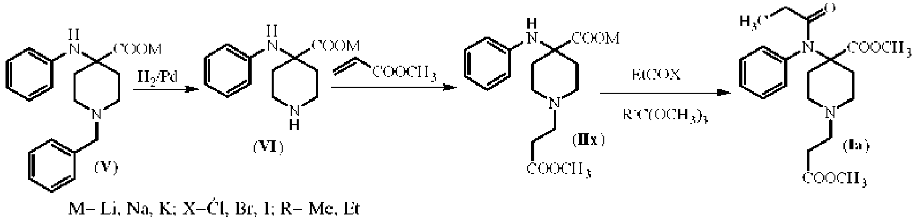

8. Verfahren nach Anspruch 7, **dadurch gekennzeichnet, dass** Benzyl - Schutzgruppe von Verbindung (V) durch Reduktion mit Wasserstoff und einem Palladium - Katalysator bei 25 bis 80 °C und einen Druck von 1-10 bar in einem aliphatischen Alkohol entfernt wird.

9. Verfahren nach Anspruch 7, **dadurch gekennzeichnet, dass** Alkylierung von Salz der 4-phenylamino-piperidin-4 Carbonsäure (VI) mit Methylacrylat bei einer Temperatur von 25 bis 80 °C in einem aliphatischen Alkohol durchgeführt ist.

10. Verfahren nach den Ansprüchen 8 und 9, **dadurch gekennzeichnet, dass** aliphatisch Alkohol Methanol, Ethanol, 1-Propanol und 2-Propanol ist.

## Revendications

1. Le processus de la préparation du composé chimique avec la formule (I): 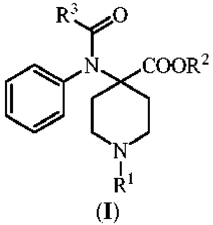 lequel comprend la réaction du composé avec la formule (II): 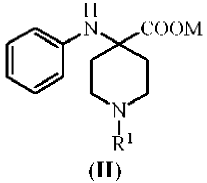 Avec l'agent d'acylation avec la formule (IV) à la présence de l'orthoester avec la formule (III) dans du solvant aprotique:
R-C(OR²)₃ (**III**)
R³-COX (**IV**)
Où les R², R³ sont indépendamment l'un de l'autre soit le méthyle soit l'éthyle; le M correspond à l'hydrogène , le lithium ou le potassium; le R correspond à l'hydrogène, le méthyle ou l'éthyle et le X correspond à l'atome d'halogène comme le chlore, le brome ou l'iode; R¹ correspond au groupe alkyle substitué ou groupe aryle substitué comme benzyle, 2-phényléthyle, 3-méthoxy-3-oxopropyle, 2-thio2furanyl-éthyle, (4-éthyle-5-oxo-4,5-dihydro-1*H*-1,2,3,4-tétrazol-1-yle)éthyle.

2. Le processus selon la revendication de brevet 1, **caractérisé par le fait que** le solvant aprotique est le toluène, tetrahydrofurane, 1,4-dioxane, dichlorométhane, acétate d'éthyle, acétate d'isopropyle, DMS, DMSO et chloroforme.

3. Le processus selon la revendication de brevet 1, **caractérisé par le fait que** l'agent d'acylation est le chlorure d'acéthyle, bromure d'acéthyle, iodure d'acéthyle, chlorure de propionyle, bromure de propionyle et iodure de propionyle.

4. Le processus selon la revendication de brevet 1, **caractérisé par le fait que** l'ortho-ester est l'orthoformiate de triméthyle, orthoformiate de triéthyle, orthoacétate de triméthyle, orthopropionate de trimétyle, orthopropionate de triéthyle.

5. Le processus selon la revendication de brevet 1, **caractérisé par le fait que** la réaction s'effectue à une température comprise entre 15 et 120 °C.

6. Le processus selon la revendication de brevet 1, **caractérisé par le fait que** le composé avec la formule (I) est isolé sous forme du sel d'addition de l'acide.

7. Le processus de la préparation du rémiphentanile **(la)** selon la figure introduite consiste aux étapes suivantes:
a) débenzylation catalytique du sel de lithium, sodium ou potassium de l'acide 1-benzyle-4-(phénylamino)piperidine-4-carboxylique (V) avec du palladium sur du charbon activé.
b) alkylation du sel de litium, sodium ou potassium de l'acide 4-phénylamino-4-piperidine carboxylique (VI) avec acrylate de méthyle
c) acylation du sel de litium, sodium ou potassium de l'acide 1-(3-méthoxy-3-oxopropyle)-4-phénylamino-4-piperidine carboxylique (**IIx**) avec du chlorure de propionyle, bromure de propionyle ou iodure de propionyle à la présence de l'orthoacétate de triméthyle ou orthopropionate de triméthyle dans dichlorométhane et le rémiphentanile résultant est isolé sous forme du sel d'oxalate ou d'hydrochlorure.

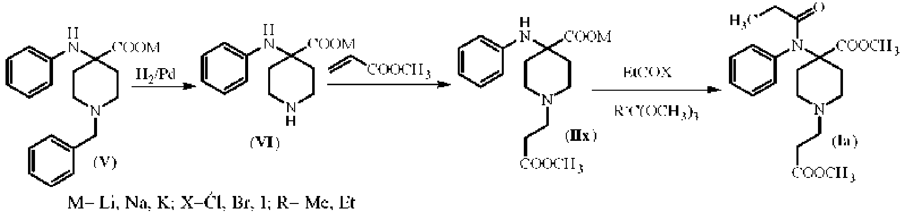

8. Le processus selon la revendication 7 **caractérisé par le fait que** le groupe protecteur benzyle est éliminé du composé (V) par réduction avec l'hydrogène et le catalyseur de palladium à une température entre 25 et 80 °C et pression 1 à 10 bars dans de l'alcool aliphatique.

9. Le processus selon la revendication 7 **caractérisé par le fait que** l'alkylation du sel de l'acide carboxylique de 4-phénylamino-4-piperidine (VI) avec l'acrylate de méthyle, s'effectue à une température entre 25 à 80 °C dans alcool aliphatique.

10. Le processus selon les revendications de brevet 8 et 9, **caractérisé par le fait que** l'alcool aliphatique est le méthanol, éthanol, 1-propanol et 2-propanol.
